# EUROPEAN PATENT APPLICATION

(11) **EP 0 758 019 A1**
(43) Date of publication of application: **12.02.1997**
(21) Application number: 95110866.1
(22) Date of filing: 12.07.1995
(51) Int. Cl.: C12N 15/11, C12N 15/10

(54) **A process for the preparation of replicators**

(71) Applicant: Max-Planck-Gesellschaft zur Förderung der Wissenschaften e.V., D-80084 München (DE)
(72) Inventor: Biebricher, Christof K., D-37139 Adelebsen (DE); Luce, Rüdiger, D-37412 Herzberg (DE)
(74) Representative: Meyers, Hans-Wilhelm, Dr.Dipl.-Chem.

(57) **Abstract**

A process for the preparation of oligo- or polynucleotides being amplified autocatalytically (replicators), wherein a polymerase in high concentrations is incubated with substrates, such as nucleotides, without external matrices under conditions by which the polymerase is able to synthesize oligo- or polynucleotides of the substrate and to replicate oligo- or polynucleotides and, optionally the formed oligo- or polynucleotides are subsequently isolated.

## Description

The present invention is concerned with a process for the preparation of replicators, in particular RNA replicators, the replicators obtainable by the process of the invention and use of the replicator of the invention for modifying viral information in the cell.

So-called "satellite viruses", smaller viruses, have been discovered which are amplified in virus-infected cells, mostly plant cells. The respective amplification takes place by the enzymes responsible for viral multiplication. For the propagation of such molecules double infection of host cells by the virus and its satellite is required. Their genetic complexity is high. Their genome sizes are more than 1000 nucleotides and their genetic origin is unknown. Also known are virusoids, naked RNA molecules that are amplified by viral replicases. Their genetic origin in cells is unknown. Viroids which are naked RNA are amplified by enzymes present in uninfected plant cells. Many viroids are symptomless, some cause mild symptoms, and a few, but easily recognizable species cause severe symptoms, e.g. stunted growth. Viroids and Virusoids need vectors for their propagation, e.g. by sucking arthropods and spread easily by vegetative propagation in commercial horticulture.

RNA viruses of the levivirus family are able to infect bacteria. Leviviridae, e.g. as best known example the coliphage Qβ, are plus stranded viruses that are parasitic mRNA coding for only few genes, among them for an enzyme called replicase that amplifies specifically the viral RNA while ignoring the host RNA. The replicases can be isolated in active form and the amplification process has thus been extensively studied in vitro.

In a surprising manner replicators can be prepared by the process of the invention. A polymerase in high concentrations is incubated with substrates of the polymerase such as nucleotides, without external matrices under conditions by which the polymerase is able to synthesize oligo- or polymers of the substrates and to replicate the oligo- or polymers which are formed. Optionally the formed oligo- or polynucleotides are subsequently isolated.

It was surprising that polymerases can synthesize oligo- or polynucleotides from nucleotides without template instruction. It seems to contradict natural philosophy: the emergence of new information virtually from the void is a rather unique phenomenon. It seems to contradict the basic natural law "Ex nihilo nihil fit" or nothing is produced from nothing. However, there is no clash of the phenomenon observed with any natural law. As described above, the information gain is obtained by evolution. Selecting products with specific properties from an unspecific random mixture of substances is now a common technique in evolutionary biotechnology and combinatorial chemistry.

The result of the present invention show how the generation of replicators can be achieved by using a general property of DNA- and RNA-dependent RNA polymerases. DNA-dependent RNA polymerases are not at all involved in vivo in RNA replication, synthesizing RNA by transcription from DNA, a central role in genetic expression. DNA-dependent RNA polymerases usually ignore RNA as a template. However, when RNA polymerases, e.g. the RNA polymerase of coliphage T7, which can be readily purified in large amounts, is incubated for extended periods of time, RNA replicators are generated. Advantageously the replicators are specific for the polymerase that generated them. For example, RNA replicators generated by T7 RNA polymerase are readily amplified by T7 RNA polymerase but not at all by Qβ replicase and vice versa. The amplification of replicators once they are generated is rapid. Small amounts of replicator RNA are amplified exponentially with an overall replication rate in the range of k = 10⁻²s⁻¹. Furthermore, when both natural DNA templates and RNA replicators are offered to the RNA polymerase, they compete one with the other initially with approximately equal chances. However, the replicator is amplified by the polymerase during the reaction while the natural DNA template is not, hence the replicator soon outcompetes the natural template.

In the context of the specification of this invention, the following terms are used in a more specific way and shall be thus defined.

*Replicators* are defined here in particular as single-stranded RNA molecules that are amplified autocatalytically by an enzyme, a DNA- or RNA-dependent RNA polymerase. A single-stranded RNA binding to the enzyme and used for replicating is defined as *template*; the RNA strand synthesized during the replication process is called a replica. Template and replica are complementary one to another.

*Autocatalytic amplification* is a process where the concentration of RNA strands and their complements increase exponentially with time. It requires that a replica produced in one replication round may serve as a template in another replication round. In this way, the original template is reproduced faithfully. Since both RNA strands are inter-changeable as template and replica, they are usually named as *plus* and *minus* strands.

*Substrate* of RNA amplification are the four natural ribonucleotide 5'-triphosphates or chemical analogues thereof.

*Replication mix* is defined as the ingredients of the solution required for the reaction to proceed, disregarding enzyme or template. It comprises substrate, a suitable buffer, salts, in particular those containing Mg²⁺, and ingredients that stabilize the enzyme in solution, e.g. glycerol, SH-protecting reagent and/or nonionic detergents.

As mentioned above according to the invention preferably polymerases such as DNA- or RNA-dependent RNA polymerases are used. Preferably the RNA polymerase is incubated for a long period of time with respect to a normal cycle period of the polymerase reaction. For example, the term normal cycle period of a RNA polymerase means for T7 RNA polymerase 50s whereas according to the invention the RNA polymerase should be incubated with its substrates for an extended period of time which is in the range of 10 hours or more. Generally better incubation times of the polymerase with the substrates are about 1000 times longer then the cycle period of the polymerase.

Further preferred is the presence of the substrates of the polymerase in relatively high concentration. This means that nucleotides are present preferably in the mM range each. As an example, the time required for generating replicators has been found with Qβ replicase to be strongly dependent on the conditions, in particular on the enzyme and substrate concentrations. At low enzyme concentrations e.g. < 100 nM, substrate concentrations < 100 µM and/or ionic strength > 100 mM, template-free generation of replicators becomes so slow that the time required is in the orders of several days. Under these conditions, templates can be amplified without possible interference of template-free synthesis. The generation of replicators functions also at lower temperatures down to the freezing point. Therefore, for the efficient generation of replicators, reaction conditions are preferred that include low ionic strengths and high concentration of substrate and enzyme.

Template-free synthesis can be speeded up by adding oligo-and polynucleotides of a specific or preferentially random composition. The reason for this observed stimulation may be that the first step has been described as the slow synthesis of random oligo- and polynucleotides and this step can be skipped when these reagents are added to the reaction mixture a priori. Oligonucleotides of a specific sequence may be preferentially elongated by nonspecific nucleotide incorporation, thereby avoiding the particularly slow nucleation step of condensing two nucleoside triphosphates.

According to the invention it is possible to select replicators directly from random sequences. It should be noted, however, what is going on is not simply an amplification of a preexisting molecule, since it is extremely unlikely that a potent replicator can be found in a random sequence mixture. Potent replicators have chain lengths of approximately 80 to 220 nucleotides and the chance to find a specific sequence in it would be smaller than 4⁻⁸⁰ (about 10⁻⁴⁸).

Small replicators have chain lengths of 30 nucleotides and the chance to find a replicatable sequence is still extremely small. Therefore, initially no RNA synthesis is observed even when large amount of random polynucleotides are added. However, after some hours (e.g. two hours) which is much longer than needed for amplification of an RNA strand by a factor of 10¹⁰ but considerably less than the 10 to 30 hours needed for selection in the absence of a template, RNA synthesis can be detected.

The initial products which are found mostly are quite inefficient replicators with chain lengths of only 30 to 40 nucleotides and often low replication efficiencies. During the amplification reaction, the replicators do not remain genetically stable, but evolve rapidly to more efficient replicators with longer sequences and higher efficiencies. As the replication efficiency increases, not only the selection pressure to evolve but also the chances to reach a higher fitness peak by a mutation decrease sharply. One might consider that the process described also takes place in the initial selection phase which can not be observed. Therefore, to some extent it can be expected that the polymerases only occasionally find a sequence that can be partially or fully replicated even though only at low efficiency. Replication at low efficiency opens the way to evolutionary optimization which rapidly leads to more and more efficient species. Therefore, even a short sequence which can be detected as the result of an oligonucleotide of polynucleotide-aided incorporation are not fundamentally different from the template-free reactions. The selection mechanism may help to explain why generation of replicators is also stimulated by RNA sequences that do not replicate. Biebricher & Luce, EMBO J., 1992 describe optimization processes in vitro. The increase in molecular weight is caused by RNA recombination processes. A replica-enzyme complex may dissociate from a template and reassociate at a similar sequence of the same or another RNA strand. Tandem or reverse duplications of sequence parts, therefore, occur which can be further optimized by normal point mutations. Among these, base deletions and insertions are relative frequent errors of RNA polymerase, particularly at clusters of repeated nucleotides.

In a preferred embodiment of the present invention the replicator is provided with a signal of viral packaging, a nucleotide sequence at least partially hybridizing with viral nucleic acid and/or nucleotides which are capable of binding with viral coat-proteins. This is advantageous because modified viruses can be obtained after application of the replicators.

The generation of replicators in vitro may require isolation of an active polymerase. From many sources, this has not yet been achieved. In particular, the RNA-dependent RNA polymerases of most animal and plant RNA viruses are notoriously difficult to isolate in an active form. However, it is not essential to work in a defined in vitro system. Late in the infection cycles, the polymerase concentrations become large enough to allow the reaction to take place in vivo, since high amounts of oligonucleotides and polynucleotides are present that can reduce the time required for generation of replicators. From levivirus-infected cells, a heterogeneous replicator fraction has been isolated (Banerjee, Eoyang, Hori & August, J. mol. Biol., 1967) that is not present in uninfected cells. Sequences data are not yet produced, but it has been speculated that this RNA has been produced from viral RNA material. However, measurements in our laboratory have shown that infection by viruses is not required for the emergence of this type of replicators; it suffices to express the polymerase, e.g., from a plasmid bearing the polymerase gene. This observation suggests that the replicators may have been generated by the same evolutionary mechanism as described for the in vitro generation.

For RNA viruses whose active polymerases can not be isolated, an alternative way is to express the polymerases in vivo to obtain high polymerase concentrations, preferentially by cloning the polymerase genes with known recombinant techniques. If cloning is not possible, one can investigate virus-infected cells late in the infection cycle directly for short replicators. Especially cells that show persistent infection should be investigated. In contrast to the in vitro generation of replicators, the isolation of replicators from the many intracellular RNA species is the main technical problem. However, in contrast to cellular RNA that is synthesized by transcription, replicators are always present in full-length plus and minus strands. This property can be used for isolation of the replicators. Under conditions that completely denature proteins, e.g. in 5 M guanidinium hydrochloride, the cellular RNA is incubated at 50°C for several hours. Plus and minus RNA strands anneal under these conditions to form double-stranded RNA. Double-stranded RNA in the expected length range of 60 - 500 can then be isolated by two-dimensional electrophoresis. From the double-stranded RNA, the single-stranded RNA replicators can be obtained by melting of the double strand. Small amounts of RNA can be cloned or amplified as cDNA by conventional methods.

In a preferred embodiment of the present invention the replicator once isolated is provided with a functional group, e.g., with a signal sequence for viral packaging, or with sequences hybridizing to a target or binding to a protein.

A particularly important function is providing the signal for viral packaging, because it opens the way of transmitting a replicator from cell to cell. Horizontal transfer of normal replicators seems not to be possible, because there is no specific mechanism of a replicator for leaving a cell and entering another. Even though there is such a mechanism for the viral RNA, the replicator can not use it, because it is not packaged. Typically a recognition site has to be introduced into the replicator that allows it to hitchhike on the viral infection cycle. Particularly good candidates for achieving this are the binding sites for viral coat proteins which have been identified for many viruses.

A replicator able to hitchhike on the viral transfer system modifies the growth of the virus substantially. In the beginning, it does not interfere with expression of the viral genome and does not produce expression products. Noxious side effects of a replicator on the cell have never been observed.

Once the virus has expressed its polymerase, the replicator starts to compete with the viral RNA for polymerase and multiplies. Depending on its success in competition, it will more or less influence viral growth. The strongest competitors will reduce viral growth so much that no viable virions are produced. In this case, neither the virus nor the replicator has a progeny. The replicators with the weakest competition are outnumbered by the viral RNA and more and more virions without replicators are produced. If the competition is optimal, virus and replicators will have progeny, but the yield of virions as well as the expression products of viral RNA in the cell is much reduced; in many cases, the infection is under good growth conditions without pathological symptoms. In mixed infections by virions without replicators and modified virions, the progeny will consist mainly of modified virions, i.e., the modified virions not only have themselves a low pathogenicity, but they also dampen a virulent infection. With more and more virus generations, produced by passaging, an optimal modification of a replicator-dampened virus population will be obtained (cf. WO 94/01545).

A viral infection may be additionally inhibited by hybridization of the viral RNA or its mRNA with at least a part of the replicator (the per se known "antisense-RNA antiviral strategy"). Another part of the replicator may contain the signal for viral packaging.

In order to interfere with viruses it is preferred that the nucleotide sequence hybridizing with viral nucleic acid is RNA.

According to the invention a process for generating a virus population which is dampening pathogenicity of a virus is described whereby a virus modified by a replicator according to the process of generating replicators according to the invention is subjected to at least two passages of infection with high viral multiplicity.

Another application of the invention of generating replicators aims at the production of RNA replicators that are capable to autonomous replication in uninfected cells. The example of viroids in plant cells show that such replicators can exist. The generation of the replicators has to be done first in vitro and follows the main strategy that has been described in this invention, but uses as polymerase one of the DNA-dependent RNA polymerases of the pertaining cells. The stringency for autonomous replication is much higher in vivo than in vitro, and it is thus necessary to work with a broad spectrum of different replicators produced in vitro.

In many cases it may be advantageous to bridge the in vitro and in vivo selection by an intermediary step, where crude or partially purified cellular extracts are used for selecting autonomously replicating RNA. In particular, it may be preferred to include a step where the in vitro incorporation is performed in the presence of RNA ligases to allow circularization of RNA. Circular RNA may be necessary to avoid exonucleolytic degradation of the RNA. In particular, the in vivo selection procedure is performed with plant cells as host. Techniques of introducing RNAs have been described: the RNA may be adsorbed to sharp-edged particles and introduced into the plant by high-speed spraying of the suspension. Furthermore, plants have the advantage that RNA molecules are easily transferred from one cell to its neighbour cells.

Once an autonomously replicating RNA is found it can be modified in such a manner that a function is introduced. Such functional areas have been inserted into the nucleotide sequence of the RNA, in particular by recombinant methods.One possible function is the presence of an exposed sequence complementary to an RNA the expression of which should be suppressed, i.e., the RNA functions as an "anti-sense RNA". Similar state of the art functions are ribozymic action on foreign RNA, for instance by forming with the target RNA a hammerhead conformation that results in a split of the target RNA. The target RNA is preferentially a viral RNA; the presence of an appropriate replicator would thus render its host cell resistant to the target virus.

Preferably RNA polymerase is used which is host specific DNA-dependent RNA polymerase. Preferably the replicator is generated in presence of ligases which are host specific in order to obtain an autonomous replicable cyclic RNA molecule having a longer persistence in the host cell.

In a preferred embodiment of the invention an autonomous replicable parasitic" RNA is obtained by administration of replicators in high concentration to host cells, which replicators are obtainable according to the method for generating replicators according to the invention. In particular, autonomous replicable parasitic RNA is used according to the invention which was at least two times passaged in the respective host cell.

In particular, the autonomous replicable parasitic RNA can be administered to plant cells.

The replicator obtainable by the process according to the invention can be used for modifying viral information in cells prior or after an infection of the cell with a virus which replicating system accepts the replicator.

The invention is described in a greater detail in the following non limiting examples.

### Examples

**Experimental Conditions for Template-free Synthesis.** The generation of replicators by template-free synthesis is dependent on the absence of replicating sequences. If as little as one replicator strand is present, it is rapidly amplified, thus preventing generation of new replicators. RNA-dependent RNA polymerases from virus-infected cells may contain endogenous RNA replicators; it is crucial that these are totally removed by the purification procedure. DNA-dependent RNA polymerases usually do not contain endogenous RNA replicators. However, once they are produced and present in a laboratory, contamination also becomes a problem. Extreme experimental precautions must therefore be taken to avoid introduction of replicators into the enzyme preparations or incorporation buffers. Once a solution has been contaminated with a replicator, there is no "sterilizing" procedure to destroy the contaminant and the solution must be discarded.

Enzymes used for amplification were purified to homogeneity from overproducing recombinant bacteria strains using virginal ion exchange columns. Purified enzymes preparations were divided into aliquots and tested for absence of amplifiable RNA by incubation of a template-free suitable incubation mixture for 4 h under conditions where template-instructed synthesis was normal, but template-free synthesis was not observed. Enzymes were pipetted with specially reserved pipets. Sterile disposable vessels, pipet tips and other equipment were used throughout. Special purity grades of chemicals were used for preparing incorporation mixes; they were always checked for absence of amplifiable RNA as described.

Preparations of the samples were performed in rooms not used for RNA analysis work. Manipulations prone to introduce contaminations were avoided.

**Template-instructed and Template-free RNA Synthesis with Qβ Replicase.** 100 µl samples Qβ buffer (50 mM tris-hydroxyaminomethan-HCl, 10 mM MgCl₂, 10% v/v glycerol, 1 mM dithiothreitol, 0.5 mM each ATP, CTP, GTP and UTP; pH 7.5) were inoculated with serial dilutions of MDV-1, an optimized RNA species of a chain length of 221 (10⁻⁸, 10⁻⁹, 10⁻¹⁰, 10⁻¹¹, 10⁻¹², 10⁻¹³, 10⁻¹⁴ corresponding to 10⁴, 10³, 10², 10, 1, < 1, << 1 RNA strand, respectively) and incubated with 3 µg Qβ replicase (150 nM S1-less enzyme) at 30°C for 20 h. 20 µl samples were withdrawn, stopped by addition of 20 µl stop mix (10 mM EDTA in formamide) and analyzed by gel electrophoresis in 12% acrylamide gels. The RNA bands in the electropherogram were stained with 0.01% ethidium bromide and visualized by UV irradiation. Inoculation with dilutions corresponding to > 100 template strand input led to defined MDV-1 RNA bands after 30 min growth. No RNA growth was detectable after 2 h at an input of < 1 strand RNA. After 20 h incubation, RNA synthesis was detected in all samples. The samples with < 1 RNA strand input contained several weak RNA bands of chain lengths of 25 - 100. The RNA band patterns of different samples were different, even though identical conditions were chosen. When 1 µl aliquots of the samples were diluted into 100 µl fresh mixtures and incubated with Qβ replicase for 30 min, growth in all tubes was observed.

With decreasing enzyme concentrations, the time required to observe synthesis in non-inoculated samples was sharply increasing. At concentrations < 80 nM replicase, no RNA synthesis was detected after 5 d in non-inoculated samples, while template-instructed synthesis was normal.

**Properties of RNA Species Produced by Template-free Synthesis.** RNA samples obtained by template-free synthesis were heterogenous in size. The sequences of the RNA components were differing from sample to sample, but similar among the RNA population of the same incubation sample. The RNA obtained was growing autocatalytically with Qβ replicase with overall rate values of 10⁻³ to 10⁻²s⁻¹, values of about 1/20 to 1/2 to those corresponding optimized templates. The RNA band patterns changed during amplification, due to an evolutionary optimization process that eventually led to selection of evolutionary stable, optimized RNA species. The RNA species contained single-stranded RNA strands complementary one to the other and double-stranded RNA. The double-stranded RNA resulted from reaction of the single-stranded RNA one with the other and was found to be inactive in replication. By serially diluting the RNA into fresh incorporation media, indefinite multiplication of the RNA was possible.

The replication process is highly selective for the template: most RNA are not replicated at all. Minimal conditions for replication have been identified, artificial RNA species that are able to replicate have been synthesized.

**Doped Template-free Synthesis with Qβ Replicase:** 100 µl samples as described above were inoculated with 100 ng crude commercial yeast RNA (Zellstoff Mannheim, Germany) and incubated with Qβ replicase as described above. The lag time required for detectable RNA synthesis was reduced by a factor of 2 to 3. Reductions of lag times by factors of ∼ 1.5 was observed by 100 ng random hexadeoxynucleotides (Boehringer Mannheim). The RNA patterns detected in electrophoresis were different from sample to sample and similar to those observed in the absence of non-replicatable templates.

**Cloning of Replicators Amplified by Qβ Replicase into DNA.** A general method has been developed to clone a replicator strand amplifiable by Qβ replicase into a DNA plasmid in such a way that large amounts of homogeneous RNA preparation of the replicator can be produced even if the replicator is not evolutionary stable. The RNA is retrotranscribed into cDNA, the cDNA is amplified and the RNA is produced by transcription. The method makes use of the short invariant sequences at both termini; therefore, the sequence or part of it need not to be known as in other procedures like the polymerase chain reaction. Furthermore, separation of the complementary single strands is not necessary and double-stranded RNA may also be used.

Limited polyadenylation was performed with highly purified poly(A) polymerase in a buffer containing: 50 mM Tris-HCl buffer (pH 7.9), 0.1 mM dithioerythritol, 0.25 NaCl, 2.5 mM MnCl₂, 50 µM ATP. 1 µg double-stranded RNA were incubated with 0.8 units highly purified poly(A) polymerase for 5 min at 37°C. Under those conditions, on average 10-12 adenyl residues were attached to the 3'termini of the RNA. The polyadenylated RNA double strands were isolated, dissolved in 40 µl H₂O, 100 pmoles p-dGAATTCTAGGGATCCATTTTTTTGGG as first strand primer were added and the RNA melted by heating at 110°C in sealed polypropylene capillaries for 90 s followed by quenching in dry ice-isopropanol mixture. 5 µl 10 x retrobuffer (final concentrations 50 mM Tris.HCl, pH 8.3; 10 mM MgCl₂; 10 mM DTT) and 2 µl 0.025 M dNTP were added, the volume adjusted to 50 µl and incubated with 25 - 50 units AMV reverse transcriptase (Stratagene) for 60 min at 42°C. The cDNA (15 - 30 % of the RNA input) was collected by phenol extraction and EtOH precipitation. The RNA template was hydrolysed by incubation in 200 µl 20% piperidin for 3 h at 50°C After lyophilization the residue was dissolved in 20 µl water and purified by EtOH precipitation and gel chromatography or gel electrophoresis. The cDNA was heated to 100°C for 1 min and rapidly cooled. 50 pmoles p-dTATAGTGAGTCGTATTAAGCTTAATACGACTCACTATAGGG, forming a double strand with protruding 3' GGG-overhangs, were added, the mixture adjusted to 50 mM Tris.HCl (pH 7.5), 10 mM MgCl₂, 1 mM DTT and 20 µM each dATP, dCTP, dGTP and dTTP in a volume of 50 µl. The mixture was incubated with 0.5 µl modified T7 DNA polymerase (Sequenase®, USB) for 10 min at 4°C and then for 10 min at 37°C. The polymerase was destroyed by heating for 15 min at 65°C and the DNA incubated for 1 h at 37°C with 5 units each of HindIII and BamHI. The DNA was collected by pheno extraction and EtOH precipitation and purified by Sepharose® 4B (Pharmacia) chromatography or gel electrophoresis. The cDNA was ligated into 200 ng BamHI/HindIII-cut vector PUC18 DNA (Amersham) by standard methods and used for transformation of competent E.coli Sure® (Stratagene) cells. Plasmids were purified from positive clones by Qiagen® columns (Qiagen, Hilden) according to the instructions of the supplier. Sequence analysis of the cloned DNA were performed by the dideoxy method using Sequenase® (USB) according the protocol of the supplier.

RNA populations of the same sequence as the strand cloned were synthesized by transcription from the plasmid. The transcription mixture contained 50 mM Tris.HCl buffer (pH 7.5), 10 mM MgCl₂, 1 mM dithiothreitol, 1.25 mM each ATP, CTP, GTP, UTP, 0.5 mg/ml plasmid DNA digested with restriction endonuclease BstXI and 50 µg/ml T7 RNA polymerase. Incubation was for 30 min at 37°C. For determination of template activity of RNA and aliquot of the transcript was used after appropriate dilution without further purification. For all other purposes, the RNA was purified by ion exchange with mono-Q® (Pharmacia) HPLC columns.

**Introducing a Coat Binding Site into a Replicating RNA.** A cDNA-clone of MNV-11, an RNA species growing optimally with Qβ replicase species, prepared as described, contained within its sequence internal NheI and Bst EII restriction sites. One sample of the clone was digested with NheI and dephosphorylated by alkaline phosphatase with standard methods. A double strand of p-dCTAGATGCTGTCTTAGACATGCAT and p-dCTAGATGCATGTCTAAGACAGCAT was ligated into the plasmid and used for transformation. Positive clones with inserts of both polarities were identified and used to synthesize RNA by transcription. An analogous construct was made from a BstEII-cut clone by ligating in p-dGTGACATGCATGTCTAAGACAGCAT and p-dGTCACATGCTGTCTTAGACATGCAT. The obtained coat protein binding site-containing recombinant RNA species was readily amplified in vitro by Qβ replicase.

**Template-free Synthesis with RNA Polymerase from Bacteriophage T7.** T7 DNA-dependent RNA polymerase was purified from an overproducing strain (Davanloo et al., 1984) by the method of Grodberg & Dunn (1987). 100 µl samples of T7 buffer (40 mM Tris.HCl, 1 mM spermidine, 10 mM MgCl₂, 5 mM dithiothreitol, 1 mM each ATP, CTP, GTP and UTP, pH 8.1) was incubated for 20 h at 37°C with 10 µg T7 RNA polymerase (1 µM). The products were analyzed by polyacrylamide gel electrophoresis. The resulting gel pattern resembles the results obtained with Qβ replicase. Serially transferring the materials in fresh T7 buffer with T7 RNA polymerase resulted in quick selection of one species; the optimized products differed from sample to sample. The optimized products grew autocatalytically with a rate of approximately 5 x 10⁻³s⁻¹. When the enzyme was saturated with template, RNA growth proceeded linearly at a rate of ∼ 2 x 10⁻³s⁻¹.

Serially transferring the template-free synthesis products into Qβ buffer and incubating with Qβ replicase did not trigger RNA growth. Trying to use a species amplifiable by Qβ replicase as template for growth with T7 RNA polymerase also failed; hence the amplifiable products are strictly specific for their proper RNA polymerase.

**Properties of RNA Species Replicated by T7 RNA Polymerase.** The replicator species contained complementary strands. One of the strand was synthesized in about 10-fold excess and arbitrarily designated as "plus strand". The 5' termini of all investigated RNA species replicated by T7 RNA polymerase began with pppGG(G). The corresponding minus strands readily formed double strands with the plus strands; their 5' ends were pppCC... Some sequences were determined. Their primary structures were not related, i. e., no consensus sequence of recognition signals was found. However, the sequences were nearly palindromic, i. e., plus and minus strands were homologous one to the other except for the termini and a short sequence in the center of the sequence. The complementary halves of the sequences were again palindromic. Two possible secondary structures with similar stability were calculated: one as a hairpin of the half length of the sequence; the other one as two hairpins of about one quarter of the sequence lengths. Digestion with single-strand-specific nucleases produced a double strand of a chain length little smaller than half length; thus the one-hairpin structure is preferred. Double-stranded RNA was unable to serve as a template for RNA polymerase. In many respects, the properties of the RNA species replicated by T7 RNA polymerase resembled to a replicating RNA species of unknown origin that was found fortuitously in a commercial T7 RNA polymerase batch (Konarska & Sharp, 1989).

When DNA with a promoter sequence (the physiological template) was present, template-free synthesis of RNA did not occur. Serial transfer of the transcription products did not lead to RNA growth. When both, the physiological template and a replicator RNA were offered, the physiological template was preferred by a factor of about 2. However, because the replicator was amplified while the physiological template was not, the replicator overgrew the physiological template and inhibited transcription.

**Template-free Synthesis with RNA Polymerase from E. coli.** RNA polymerase from E. coli was purified according to standard methods to homogeneity. 100 µl samples of RNA-polymerase buffer (50 mM Tris.HCl, 10 mM MgCl₂, 5 mM dithiothreitol, 1 mM each ATP, CTP, GTP and UTP, pH 8.1) were incubated for 20 h at 37°C with 20 µg E. coli RNA polymerase (0.5 µM). The products were analyzed by gel electrophoresis. Positive samples were amplified by serial transfers into fresh mixtures as described previously. Defined RNA species with chain lengths of 60 - 120 were found. RNA species replicated by T7 RNA polymerase were not amplified by E. coli RNA polymerase and vice versa.

## Claims

1. A process for the preparation of oligo- or polynucleotides being amplified autocatalytically (replicators), wherein a polymerase in high concentrations is incubated with substrates, such as nucleotides, without external matrices under conditions by which the polymerase is able to synthesize oligo- or polynucleotides of the substrate and to replicate oligo- or polynucleotides and, optionally the formed oligo- or polynucleotides are subsequently isolated.

2. The process according to claim 1, wherein the polymerase is DNA or RNA dependent RNA-polymerase.

3. The process according to claim 1 or 2, wherein the polymerase is incubated for a long period of time with respect to a normal cycle period of the polymerase reaction.

4. The process of claim 3, wherein the incubation time is about 1,000 times longer than the cycle period of the polymerase used.

5. The process of any one of the claims 1 to 4, wherein the substrate concentration is high.

6. The process of any one of the claims 1 to 5, wherein the reaction of the polymerase with its substrates is accelerated by addition of oligonucleotides having per se known sequence-motives and/or having stochastical sequences.

7. The process of any one of the claims 1 to 6, wherein the replicators according to one of the foregoing claims are optimized by evolutionary methods.

8. The process of any one of the claims 1 to 7, wherein the replicator is provided with a signal of viral packaging, a nucleotide sequence at least partially hybridizing with viral nucleic acid and/or nucleotides which are capable of binding with viral coat-proteins in order to obtain modified viruses after application of the replicators.

9. The process of claim 8, wherein the signal of viral packaging, the nucleotide sequence at least partially hybridizing with viral nucleic acid and/or nucleotides which are capable of binding with viral coat-proteins are combined with the replicator's nucleotide sequence by means of recombinant methods.

10. The process of claim 8 and/or 9, wherein the nucleotide sequence hybridizing with viral nucleic acid and/or nucleotides is RNA.

11. The process of claims 8 and/or 9, wherein the replicators being capable to bind to viral coat-protein are obtained from a mixture of replicators manufactured according to any one of the claims 1 to 7.

12. A process for generating a virus population which is dampening pathogenicity of a virus whereby a virus modified by a replicator according to any one of claims 8 to 11 is subjected to at least two passages of infection with high viral multiplicity.

13. The process of any one of claims 2 to 7 wherein the RNA polymerase is a host specific DNA-dependent RNA polymerase.

14. The process of claim 13, wherein the replicator is generated in presence of ligases which are host specific in order to obtain an autonomous replicable cyclic RNA having a longer persistence in the host cell.

15. The process of claim 13 and/or 14, wherein an autonomous replicable parasitic RNA is obtained by administration of replicators in high concentration to host cells, which replicators are obtainable according to any one of the claims 1 to 7 and/or 13 or 14.

16. The process of claim 15, wherein the autonomous replicable parasitic RNA is subjected to at least two passages in the respective host cell.

17. The process of claim 15, wherein the autonomous replicable parasitic RNA is administered to plant cells.

18. A process for generating autonomous replicable RNA according to any one of the claims 15 to 17, the RNA having functional areas, such as anti-sense RNA, ribozymic RNA, which have been inserted into the nucleotide sequence of the RNA with recombinant methods.

19. A replicator obtainable by a process according to any one of the claims 1 to 18.

20. Use of the replicators according to claim 19 for modifying viral information in a cell prior or after infection of the cell with a virus the replicating system of which accepts the replicator.
